# FASCICULE DE BREVET EUROPEEN

(11) **EP 1 183 368 B1**
(45) Date de publication et mention de la délivrance du brevet: **18.04.2012**
(21) Numéro de dépôt: 00938875.2
(22) Date de dépôt: 31.05.2000
(51) Int. Cl.: C12N 15/37, A61K 39/12, C07K 16/08, C07K 14/025

(54) **FRAGMENTS PROTEIQUES POLYEPITOPIQUES DES PROTEINES E6 ET E7 DE HPV, LEUR OBTENTION ET LEURS UTILIATIONS NOTAMMENT EN VACCINATION**
PROTEINFRAGMENTE DIE POLYEPITOPE DER PROTEINE E6 UND E7 VON HPV UMFASSEN, DEREN HERSTELLUNG UND VERWENDUNG INSBESONDERE ZUR IMPFUNG
PROTEINFRAGMENTE DIE VERSCHIEDENE EPITOPE DER HPV PROTEINE E6 UND E7 UMFASSEN, IHRE HERSTELLUNG UND VERWENDUNGEN INSBESONDERE ZUR IMPFUNG

(30) Priorité: 03.06.1999 FR 9907012
(43) Date de publication de la demande: 06.03.2002
(60) Demande divisionnaire: 10177363.8
(73) Titulaire: Assistance Publique - Hôpitaux de Paris, 75100 Paris (FR); INSTITUT NATIONAL DE LA SANTE ET DE LA RECHERCHE MEDICALE (INSERM), 75654 Paris Cédex 13 (FR)
(72) Inventeur: CHOPPIN, Jeannine, 93110 ROSNY SOUS BOIS (FR); BOURGAULT VILLADA, Isabelle, F-75007 Paris (FR); GUILLET, Jean-Gérard, F-75009 Paris (FR); CONNAN, Francine, F-95170 Deuil-la-Barre (FR); FERRIES, Estelle, F-75013 Paris (FR)
(74) Mandataire: Swinkels, Bart Willem
(86) Numéro de dépôt international: PCT/FR2000/001513
(87) Numéro de publication internationale: WO 2000/075336

(56) Documents cités:
- EP-A- 0 253 325
- EP-A- 0 257 754
- EP-A- 0 375 555
- EP-A- 0 451 550
- EP-A- 0 451 550
- EP-A- 0 523 391
- EP-A- 0 523 391
- WO-A-91/18294
- WO-A-92/10513
- WO-A-93/22338
- WO-A-93/22338
- WO-A-95/22317
- WO-A-97/41440
- WO-A-98/23635
- WO-A-98/23635
- WO-A-99/18995
- WO-A-99/27954
- MÜLLER M ET AL: "IDENTIFICATION OF SEROREACTIVE REGIONS OF THE HUMAN PAPILLOMAVIRUS TYPE 16 PROTEINS E4, E6, E7 AND L1" JOURNAL OF GENERAL VIROLOGY,GB,SOCIETY FOR GENERAL MICROBIOLOGY, READING, vol. 71, 1 janvier 1990 (1990-01-01), pages 2709-2717, XP000318453 ISSN: 0022-1317
- SHALLY M ET AL: "THE E6 VARIANT PROTEINS E6I-E6IV OF HUMAN PAPILLOMAVIRUS 16: EXPRESSION IN CELL FREE SYSTEMS AND BACTERIA AND STUDY OF THEIR INTERACTION WITH P53" VIRUS RESEARCH,NL,AMSTERDAM, vol. 42, 1996, pages 81-96, XP000198411 ISSN: 0168-1702
- DILLNER JOAKIM: "Enzyme immunoassay detection of induction of MHC class I expression by synthetic peptides from the E6 and E7 regions of human papillomavirus type 16" JOURNAL OF IMMUNOLOGICAL METHODS, ELSEVIER SCIENCE PUBLISHERS B.V.,AMSTERDAM, NL, vol. 167, no. 1-2, 1994, pages 195-205, XP002956559 ISSN: 0022-1759
- TINDLE R W ET AL: "A PUBLIC T-HELPER EPITOPE OF THE E7 TRANSFORMING PROTEIN OF HUMAN PAPILLOMAVIRUS 16 PROVIDES COGNATE HELP FOR SEVERAL E7 B-CELL EPITOPES FROM CERVICAL CANCER-ASSOCIATED HUMAN PAPILLOMAVIRUS GENOTYPES" juillet 1991 (1991-07) , PROCEEDINGS OF THE NATIONAL ACADEMY OF SCIENCES OF THE UNITED STATES OF AMERICA, VOL. 88, NR. 13, PAGE 5887-5891 XP002290374 ISSN: 0027-8424 * tableau 2 *
- MÜLLER M ET AL: "IDENTIFICATION OF SEROREACTIVE REGIONS OF THE HUMAN PAPILLOMAVIRUS TYPE 16 PROTEINS E4, E6, E7 AND L1" JOURNAL OF GENERAL VIROLOGY,GB,SOCIETY FOR GENERAL MICROBIOLOGY, READING, vol. 71, 1 janvier 1990 (1990-01-01), pages 2709-2717, XP000318453 ISSN: 0022-1317
- SHALLY M ET AL: "THE E6 VARIANT PROTEINS E6I-E6IV OF HUMAN PAPILLOMAVIRUS 16: EXPRESSION IN CELL FREE SYSTEMS AND BACTERIA AND STUDY OF THEIR INTERACTION WITH P53" VIRUS RESEARCH,NL,AMSTERDAM, vol. 42, 1996, pages 81-96, XP000198411 ISSN: 0168-1702

### LISTE DE SEQUENCES

<110> BIOVECTOR THERAPEUTICS INSERM
<120> FRAGMENTS PROTEIQUES POLYEPITOPIQUES DE LA PROTEINE E6 OU E7 DE HPV, LEUR OBTENTION ET LEURS UTILISATIONS NOTAMMENT EN VACCINATION
<130> WOB EPIT 2
<140>
   <141>
<150> FR 9907012
   <151> 1999-06-03
<160> 18
<170> PatentIn Ver. 2.1
<210> 1
   <211> 477
   <212> ADN
   <213> Papillomavirus humain
<220>
   <221> CDS
   <222> (1) .. (477)
<400> 1
   atg cac caa aag aga act gca atg ttt cag gac cca cag gag cga ccc    48
   Met His Gln Lys Arg Thr Ala Met Phe Gln Asp Pro Gln Glu Arg Pro
    1               5                  10                  15
   aga aag tta cca cag tta tgc aca gag ctg caa aca act ata cat gat    96
   Arg Lys Leu Pro Gln Leu Cys Thr Glu Leu Gln Thr Thr Ile His Asp
               20                  25                  30
   ata ata tta gaa tgt gtg tac tgc aag caa cag tta ctg cga cgt gag   144
   Ile Ile Leu Glu Cys Val Tyr Cys Lys Gln Gln Leu Leu Arg Arg Glu
           35                  40                  45
   gta tat gac ttt gct ttt cgg gat tta tgc ata gta tat aga gat ggg   192
   Val Tyr Asp Phe Ala Phe Arg Asp Leu Cys Ile Val Tyr Arg Asp Gly
       50                  55                  60
   aat cca tat gct gta tgt gat aaa tgt tta aag ttt tat tct aaa att   240
   Asn Pro Tyr Ala Val Cys Asp Lys Cys Leu Lys Phe Tyr Ser Lys Ile
   65                  70                  75                  80
   agt gag tat aga cat tat tgt tat agt ttg tat gga aca aca tta gaa   288
   Ser Glu Tyr Arg His Tyr Cys Tyr Ser Leu Tyr Gly Thr Thr Leu Glu
                   85                  90                  95
   cag caa tac aac aaa ccg ttg tgt gat ttg tta att agg tgt att aac   336
   Gln Gln Tyr Asn Lys Pro Leu Cys Asp Leu Leu Ile Arg Cys Ile Asn
               100                 105                 110
   tgt caa aag cca ctg tgt cct gaa gaa aag caa aga cat ctg gac aaa   384
   Cys Gln Lys Pro Leu Cys Pro Glu Glu Lys Gln Arg His Leu Asp Lys
           115                 120                 125
   aag caa aga ttc cat aat ata agg ggt cgg tgg acc ggt cga tgt atg   432
   Lys Gln Arg Phe His Asn Ile Arg Gly Arg Trp Thr Gly Arg Cys Met
       130                 135                 140
   tct tgt tgc aga tca tca aga aca cgt aga gaa acc cag ctg tga       477
   Ser Cys Cys Arg Ser Ser Arg Thr Arg Arg Glu Thr Gln Leu
   145                 150                 155
<210> 2
   <211> 158
   <212> PRT
   <213> Papillomavirus humain
<400> 2
   Met His Gln Lys Arg Thr Ala Met Phe Gln Asp Pro Gln Glu Arg Pro
     1               5                  10                  15
   Arg Lys Leu Pro Gln Leu Cys Thr Glu Leu Gln Thr Thr Ile His Asp
               20                  25                  30
   Ile Ile Leu Glu Cys Val Tyr Cys Lys Gln Gln Leu Leu Arg Arg Glu
           35                  40                  45
   Val Tyr Asp Phe Ala Phe Arg Asp Leu Cys Ile Val Tyr Arg Asp Gly
       50                  55                  60
   Asn Pro Tyr Ala Val Cys Asp Lys Cys Leu Lys Phe Tyr Ser Lys Ile
    65                  70                  75                  80
   Ser Glu Tyr Arg His Tyr Cys Tyr Ser Leu Tyr Gly Thr Thr Leu Glu
                    85                  90                  95
   Gln Gln Tyr Asn Lys Pro Leu Cys Asp Leu Leu Ile Arg Cys Ile Asn
               100                 105                 110
   Cys Gln Lys Pro Leu Cys Pro Glu Glu Lys Gln Arg His Leu Asp Lys
           115                 120                 125
   Lys Gln Arg Phe His Asn Ile Arg Gly Arg Trp Thr Gly Arg Cys Met
       130                 135                 140
   Ser Cys Cys Arg Ser Ser Arg Thr Arg Arg Glu Thr Gln Leu
   145                 150                 155
<210> 3
   <211> 90
   <212> ADN
   <213> Séquence artificielle
<220>
   <223> Description de la séquence artificielle: fragment de la séquence codant pour E6 de HPV et séquence peptidique correspondante
<220>
   <221> CDS
   <222> (1) .. (90)
<400> 3
   cga ccc aga aag tta cca cag tta tgc aca gag ctg caa aca act ata    48
   Arg Pro Arg Lys Leu Pro Gln Leu Cys Thr Glu Leu Gln Thr Thr Ile
     1               5                  10                  15
   cat gat ata ata tta gaa tgt gtg tac tgc aag caa cag tta            90
   His Asp Ile Ile Leu Glu Cys Val Tyr Cys Lys Gln Gln Leu
               20                  25                  30
<210> 4
   <211> 30
   <212> PRT
   <213> Séquence artificielle
<223> Description de la séquence artificielle: fragment de la séquence codant pour E6 de HPV et séquence peptidique correspondante
<400> 4
   Arg Pro Arg Lys Leu Pro Gln Leu Cys Thr Glu Leu Gln Thr Thr Ile
     1               5                  10                  15
   His Asp Ile Ile Leu Glu Cys Val Tyr Cys Lys Gln Gln Leu
                20                  25                  30
<210> 5
   <211> 66
   <212> ADN
   <213> Séquence artificielle
<220>
   <223> Description de la séquence artificielle: fragment de la séquence codant pour E6 de HPV et séquence peptidique correspondante
<220>
   <221> CDS
   <2.22> (1) .. (66)
<400> 5
   cga cgt gag gta tat gac ttt gct ttt cgg gat tta tgc ata gta tat    48
   Arg Arg Glu Val Tyr Asp Phe Ala Phe Arg Asp Leu Cys Ile Val Tyr
     1               5                  10                  15
   aga gat ggg aat cca tat                                            66
   Arg Asp Gly Asn Pro Tyr
                20
<210> 6
   <211> 22
   <212> PRT
   <213> Séquence artificielle
<223> Description de la séquence artificielle: fragment de la séquence codant pour E6 de HPV et séquence peptidique correspondante
<400> 6
   Arg Arg Glu Val Tyr Asp Phe Ala Phe Arg Asp Leu Cys Ile Val Tyr
     1               5                  10                  15
   Arg Asp Gly Asn Pro Tyr
                20
<210> 7
   <211> 87
   <212> AD::
   <213> Sequence artificielle
<220>
   <223> Description de la séquence artificielle: fragment de la sequence codant pour E6 de HPV et séquence peptidique correspondante
<220>
   <221> CDS
   <222> (1)..(87)
<400> 7
   att agt gag tat aga cat tat tgt tat agt ttg tat gga aca aca tta   48
   Ile Ser Glu Tyr Arg His Tyr Cys Tyr Ser Leu Tyr Gly Thr Thr Leu
     1               5                  10                  15
   gaa cag caa tac aac aaa ccg ttg tgt gat ttg tta att               87
   Glu Gln Gln Tyr Asn Lys Pro Leu Cys Asp Leu Leu Ile
                20                  25
<210> 8
   <211> 29
   <212> PRT
   <213> Séquence artificielle
<223> Description de la séquence artificielle: fragment de la séquence codant pour E6 de HPV et séquence peptidique correspondante
<400> 8
   Ile Ser Glu Tyr Arg His Tyr Cys Tyr Ser Leu Tyr Gly Thr Thr Leu
     1               5                  10                  15
   Glu Gln Gln Tyr Asn Lys Pro Leu Cys Asp Leu Leu Ile
                20                  25
<210> 9
   <211> 66
   <212> ADN
   <213> Séquence artificielle
<220>
   <223> Description de la séquence artificielle: fragment de la séquence codant pour E6 de HPV et séquence peptidique correspondante
<220>
   <221> CDS
   <222> (1)..(66)
<400> 9
   tgt cct gaa gaa aag caa aga cat ctg gac aaa aag caa aga ttc cat   48
   Cys Pro Glu Glu Lys Gln Arg His Leu Asp Lys Lys Gln Arg Phe His
     1               5                  10                  15
   aat ata agg ggt cgg tgg                                           66
   Asn Ile Arg Gly Arg Trp
                20
<210> 10
   <211> 22
   <212> PRT
   <213> Séquence artificielle
<223> Description de la séquence artificielle: fragment de la séquence codant pour E6 de HPV et séquence peptidique correspondante
<400> 10
   Cys Pro Glu Glu Lys Gln Arg His Leu Asp Lys Lys Gln Arg Phe His
     1               5                  10                  15
   Asn Ile Arg Gly Arg Trp
                   20
<210> 11
   <211> 297
   <212> ADN
   <213> Papillomavirus humain
<220>
   <221> CDS
   <222> (1)..(297)
<400> 11
   atg cat gga gat aca cct aca ttg cat gaa tat atg tta gat ttg caa    48
   Met His Gly Asp Thr Pro Thr Leu His Glu Tyr Met Leu Asp Leu Gln
     1               5                  10                  15
   cca gag aca act gat ctc tac tgt tat gag caa tta aat gac agc tca    96
   Pro Glu Thr Thr Asp Leu Tyr Cys Tyr Glu Gln Leu Asn Asp Ser Ser
                20                  25                  30
   gag gag gag gat gaa ata gat ggt cca gct gga caa gca gaa ccg gac   144
   Glu Glu Glu Asp Glu Ile Asp Gly Pro Ala Gly Gln Ala Glu Pro Asp
            35                  40                  45
   aga gcc cat tac aat att gta acc ttt tgt tgc aag tgt gac tct acg   192
   Arg Ala His Tyr Asn Ile Val Thr Phe Cys Cys Lys Cys Asp Ser Thr
        50                  55                  60
   ctt cgg ttg tgc gta caa agc aca cac gta gac att cgt act ttg gaa   240
   Leu Arg Leu Cys Val Gln Ser Thr His Val Asp Ile Arg Thr Leu Glu
    65                  70                  75                  80
   gac ctg tta atg ggc aca cta gga att gtg tgc ccc atc tgt tct cag   288
   Asp Leu Leu Met Gly Thr Leu Gly Ile Val Cys Pro Ile Cys Ser Gln
                    85                  90                  95
   aaa cca taa                                                       297
   Lys Pro
<210> 12
   <211> 98
   <212> PRT
   <213> Papillomavirus humain
<400> 12
   Met His Gly Asp Thr Pro Thr Leu His Glu Tyr Met Leu Asp Leu Gln
   1               5                   10                  15
   Pro Glu Thr Thr Asp Leu Tyr Cys Tyr Glu Gln Leu Asn Asp Ser Ser
               20                  25                  30
   Glu Glu Glu Asp Glu Ile Asp Gly Pro Ala Gly Gln Ala Glu Pro Asp
           35                  40                  45
   Arg Ala His Tyr Asn Ile Val Thr Phe Cys Cys Lys Cys Asp Ser Thr
       50                  55                  60
   Leu Arg Leu Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Lys Pro
<210> 13
   <211> 69
   <212> ADN
   <213> Séquence artificielle
<220>
   <223> Description de la séquence artificielle: fragment de la séquence codant pour E7 de HPV et séquence peptidique correspondante
<220>
   <221> CDS
   <222> (1)..(69)
<400> 13
   gga gat aca cct aca ttg cat gaa tat atg tta gat ttg caa cca gag    48
   Gly Asp Thr Pro Thr Leu His Glu Tyr Met Leu Asp Leu Gln Pro Glu
   1               5                   10                  15
   aca act gat ctc tac tgt tat                                        69
   Thr Thr Asp Leu Tyr Cys Tyr
               20
<210> 14
   <211> 23
   <212> PRT
   <213> Séquence artificielle
<223> Description de la séquence artificielle: fragment de la séquence codant pour E7 de HPV et séquence peptidique correspondante
<400> 14
   Gly Asp Thr Pro Thr Leu His Glu Tyr Met Leu Asp Leu Gln Pro Glu
     1               5                  10                  15
   Thr Thr Asp Leu Tyr Cys Tyr
                20
<210> 15
   <211> 51
   <212> ADN
   <213> Séquence artificielle
<220>
   <223> Description de la séquence artificielle: fragment de la séquence codant pour E7 de HPV et séquence peptidique correspondante
<220>
   <221> CDS
   <222> (1) .. (51)
<400> 15
   caa gca gaa ccg gac aga gcc cat tac aat att gta acc ttt tgt tgc    48
   Gln Ala Glu Pro Asp Arg Ala His Tyr Asn Ile Val Thr Phe Cys Cys
     1               5                  10                  15
   aag                                                                51
   Lys
<210> 16
   <211> 17
   <212> PRT
   <213> Séquence artificielle
<223> Description de la séquence artificielle: fragment de la séquence codant pour E7 de HPV et séquence peptidique correspondante
<400> 16
   Gln Ala Glu Pro Asp Arg Ala His Tyr Asn Ile Val Thr Phe Cys Cys
     1               5                  10                  15
   Lys
<210> 17
   <211> 57
   <212> ADN
   <213> Séquence artificielle
<220>
   <223> Description de la séquence artificielle: fragment de la séquence codant pour E7 de HPV et séquence peptidique correspondante
<220>
   <221> CDS
   <222> (1) .. (57)
<400> 17
   ttg gaa gac ctg tta atg ggc aca cta gga att gtg tgc ccc atc tgt   48
   Leu Glu Asp Leu Leu Met Gly Thr Leu Gly Ile Val Cys Pro Ile Cys
    1               5                  10                  15
   tct cag aaa                                                        57
   Ser Gln Lys
<210> 18
   <211> 19
   <212> PRT
   <213> Séquence artificielle
<223> Description de la séquence artificielle: fragment de la séquence codant pour E7 de HPV et séquence peptidique correspondante
<400> 18
   Leu Glu Asp Leu Leu Met Gly Thr Leu Gly Ile Val Cys Pro Ile Cys
    1               5                  10                  15
   Ser Gln Lys

## Description

La présente invention a pour objet des fragments protéiques polyépitopiques, tels que ceux de la protéine E6 des papillomavirus humains, leur procédé d'obtention, et leurs utilisations, notamment dans le domaine de la vaccination thérapeutique ou préventive.

L'invention a plus particulièrement pour objet l'utilisation de fragments polyépitopiques d'une protéine déterminée pour la préparation de médicaments destinés à la prévention ou au traitement de pathologies dans lesquelles ladite protéine est reconnue par le système immunitaire cellulaire.

Avantageusement, lesdits fragments polyépitopiques sont tels que leur acide aminé N-terminal correspond à l'acide aminé N-terminal de l'épitope situé en amont d'un ou plusieurs autres épitopes d'une région polyépitopique de ladite protéine, et leur acide aminé C-terminal correspond à l'acide aminé C-terminal de l'épitope situé en aval du ou des épitopes susmentionnés de ladite région polyépitopique.

Ainsi, les fragments protéiques polyépitopiques susmentionnés de la présente invention correspondent avantageusement aux régions polyépitopiques d'une protéine déterminée, à savoir aux régions contenant plusieurs épitopes reconnus par les cellules T en association avec les différentes molécules du complexe majeur d'histocompatibilité (CMH), lesdites régions étant sélectionnées parmi celles ayant la caractéristique d'être dégradées *in vitro* en peptides plus courts par des protéasomes, tel que le protéasome 20S, lorsque le fragment protéique testé est mis en présence dudit protéasome, notamment selon la méthode détaillée suivante. Le fragment protéique (environ 75 µg lorsqu'il s'agit d'un polypeptide d'environ 30 acides aminés) est incubé à 37°C avec environ 15µg de protéasome 20S (Calbiochem Ref 539150, La Jolla, CA, USA) dans 500 µl du tampon suivant : 20 mM Tris-HCl pH8, 0,5 mM EDTA. Des aliquots de 50 µl sont prélevés après des temps d'incubation de 24 et 48 heures, et sont analysés par chromatographie liquide haute pression (HPLC). Les produits de digestion des protéasomes sont séparés par RP-HPLC (Perkin Elmer) en utilisant une colonne C18 et un gradient acétonitrile (de 0 à 100 % contenant 0,1 % d'acide trifluoroacétique, en 90 mn, taux d'élution 0,8 ml/mn). Les produits de clivage sont détectés à 214 nm par un détecteur à absorption (759A, Applied Biosystems).

Avantageusement les régions polyépitopiques définies ci-dessus possèdent la caractéristique de contenir des acides aminés hydrophobes.

Les différents épitopes de la région polyépitopique de la protéine déterminée, et délimitant les fragments protéiques polyépitopiques, sont avantageusement sélectionnés parmi les peptides ;
- se liant à une molécule déterminée du CMH, notamment à une molécule de type HLA déterminé, et ce jusqu'à des concentrations d'environ 10⁻⁶ M à environ 10⁻¹⁰ M en peptide pour des concentrations d'environ 10⁻⁷ M en molécule HLA, notamment dans les conditions décrites ci-après,
- et formant un complexe stable avec cette molécule du CMH, à savoir notamment un complexe dans lequel ledit peptide reste lié à ladite molécule pendant au moins environ 3 heures à 37°C.

A titre d'illustration, les épitopes susmentionnés de l'invention sont sélectionnés parmi les peptides susceptibles :
- d'une part de s'associer avec les molécules du CMH, notamment par mise en oeuvre de la méthode suivante :
   - incubation (notamment pendant environ 2 heures à 25°C, puis environ 15 heures à 4°C) du peptide en présence de molécules du CMH, provenant de la lyse de cellules humaines ou animales, ou purifiées notamment par chromatographie d'affinité à partir de lignées cellulaires humaines ou animales,
   - piégeage des complexes formés lors de l'étape précédente sur un support solide recouvert d'un premier anticorps, notamment monoclonal, reconnaissant spécifiquement les molécules du CMH dans leur conformation dépendante de leur liaison audit peptide,
   - addition sur le support solide précédent d'un deuxième anticorps marqué, notamment par couplage à un marqueur radioactif, enzymatique ou fluorescent, ledit anticorps marqué reconnaissant spécifiquement soit les chaînes lourdes du CMH dans leur conformation dépendante de leur liaison au peptide, soit la chaîne légère du CMH ou la β2-microglobuline se liant spécifiquement aux différentes chaînes lourdes du CMH dans leur conformation susmentionnée,
   - détection, après rinçage du support solide, de l'éventuelle présence du deuxième anticorps marqué resté fixé sur le support solide, témoignant d'un effet d'association entre les molécules du CMH et le peptide étudié,
- et, d'autre part, de former un complexe avec lesdites molécules du CMH, dont la stabilité peut être évaluée par mise en oeuvre d'une méthode de suivi dans le temps de la liaison établie entre le peptide et les molécules du CMH, cette méthode étant avantageusement effectuée selon un protocole identique à la méthode précédente, mais dans laquelle l'étape d'incubation du peptide en présence des molécules du CMH sur le support solide recouvert dudit premier anticorps, est précédée par une étape préalable d'élimination du peptide libre susceptible d'être présent dans le milieu réactionnel, notamment par lavage du support solide, ladite étape d'incubation étant effectuée (avantageusement à une température de 37°C) pendant des temps variables de 1h, 3h, 5h, 24h et 48h.

Comme mentionné ci-dessus, les épitopes de l'invention doivent être reconnus par les cellules T en association avec les molécules du CMH et s'associer à ces dernières, notamment dans le cadre de la mise en oeuvre du test de reconnaissance décrit ci-dessus. Cette association peut être faible (détectable à des concentrations en analogues peptidiques de l'ordre de 10⁻⁴ à 10⁻⁵ M), intermédiaire (détectable à des concentrations en analogues peptidiques de l'ordre de 10⁻⁶ à 10⁻⁷ M), ou forte (détectable à des concentrations en analogues peptidiques de l'ordre de 10⁻⁸ à 10⁻⁹ M). Les peptides associés aux molécules du CMH dans le cadre de la présente invention sont de préférence susceptibles de se lier pendant au moins environ 3 heures auxdites molécules du CMH.

L'invention a plus particulièrement pour objet les épitopes (encore désignés peptides ci-dessus et ci-après) tels que décrits ci-dessus et caractérisés en ce qu'ils sont sélectionnés parmi ceux susceptibles :
- d'induire *in vitro* la cytolyse par des lymphocytes T cytotoxiques, de cellules cibles présentant à leur surface le peptide susmentionné associé aux molécules du CMH, lesdits lymphocytes T cytotoxiques étant avantageusement prélevés sur un patient atteint d'une pathologie dans laquelle est impliqué le peptide étudié,
- et d'induire *in vitro* la sécrétion de cytokines (ou interleukines) par les lymphocytes T cytotoxiques susmentionnés, notamment IL-2, IL-4 ou l'interféron y.

Le cas échéant, les épitopes susmentionnés sont choisis parmi ceux capables d'induire *in vitro* l'apparition et la croissance de lymphocytes T cytotoxiques à partir de cellules humaines ou animales, notamment à partir de cellules mononucléées issues du sang périphérique (PBMC), en présence de facteurs nécessaires à la croissance et la différenciation des cellules T cytotoxiques.

Les fragments protéiques polyépitopiques de l'invention sont davantage caractérisés en ce qu'ils sont susceptibles de contenir des épitopes CD4 reconnus par les cellules T auxiliaires en association avec les molécules du CMH de classe II, cette propriété favorisant l'induction et le maintien des cellules T CD8⁺ reconnaissant les épitopes compris dans lesdits fragments.

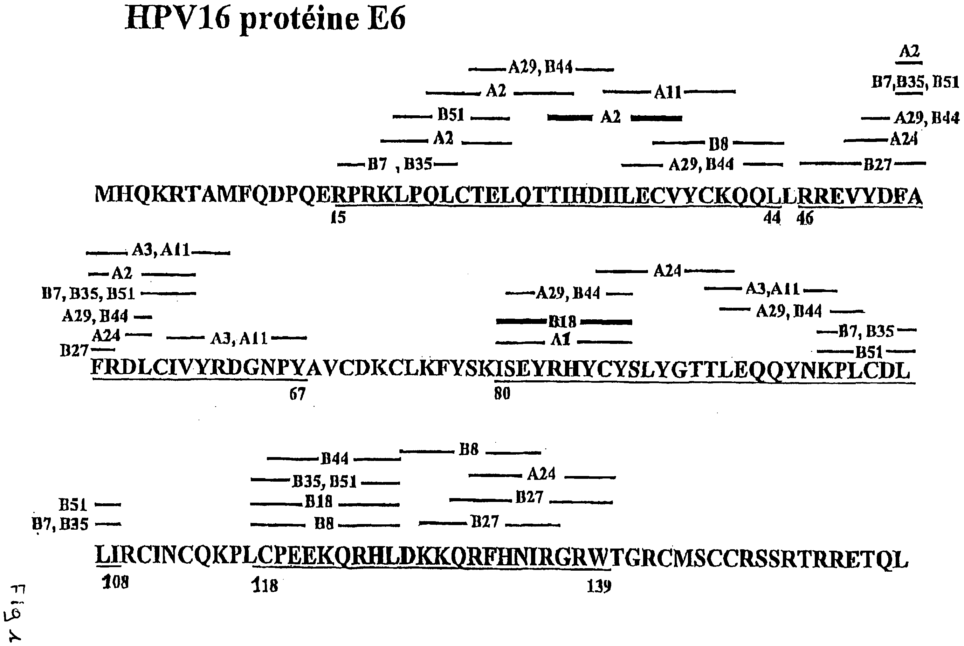

La présente invention est illustrée à l'aide de la figure 1 représentant la séquence peptidique de la protéine E6 de la souche 16 des papillomavirus humains (HPV 16), ainsi que les fragments polyépitopiques de l'invention, et les épitopes au sein de ces fragments.

L'invention a plus particulièrement pour objet les fragments polyépitopiques de la protéine E6 d'HPV, et plus particulièrement ceux de la protéine E6 représentée sur la figure 1, ou par SEQ ID NO : 2, de HPV 16, caractérisés en ce qu'ils comprennent une séquence peptidique d'environ 15 à environ 30 acides aminés, cette séquence peptidique contenant les séquences en acides aminés d'au moins 3 épitopes différents, et de préférence d'au moins 4 épitopes différents se liant de façon stable à des molécules HLA de type identique ou différent, lorsque ces épitopes sont obtenus par dégradation enzymatique de ladite séquence peptidique, notamment dans le protéasome, de sorte qu'au moins 4 molécules HLA de différents types, et de préférence au moins 5 molécules HLA de différents types, se lient à ces épitopes, ces 4 ou 5 molécules HLA étant choisies parmi celles de type A1, A2, A3, A11, A24, A29, B7, B8, B18, B27, B35, B44, B51, et B62.

Avantageusement, les fragments polyépitopiques selon invention sont tels que le nombre d'acides aminés de leur séquence peptidique est supérieur ou égal à 17, et inférieur ou égal à 30.

L'invention concerne plus particulièrement les fragments polyépitopiques de la protéine E6 de HPV définis ci-dessus, caractérisés en ce qu'ils comprennent une séquence peptidique d'environ 15 à 30 acides aminés, cette séquence peptidique contenant les séquences en acides aminés d'au moins 6 épitopes différents se liant de façon stable à des molécules HLA de type identique ou différent, lorsque ces épitopes sont obtenus par dégradation enzymatique de ladite séquence peptidique, notamment dans le protéasome, de sorte qu'au moins 6 molécules HLA de différents types, et de préférence au moins 7 molécules HLA de différents types se lient à ces épitopes, ces 6 ou 7 molécules HLA étant choisies parmi celles de type A1, A2, A3, A11, A24, A29, B7, B8, B18, B27, B35, B44, et B51.

Avantageusement, les fragments polyépitopiques de la protéine E6 selon l'invention, sont tels que le nombre d'acides aminés de leur séquence peptidique est supérieur ou égal à 20 (de préférence supérieur ou égal à 22), et inférieur ou égal à 30.

Avantageusement encore, les fragments polyépitopiques susmentionnés de la protéine E6 de HPV, sont caractérisés en ce qu'ils comprennent tous un épitope se liant à la molécule HLA de type B35, un épitope se liant à la molécule HLA de type B44, et un épitope se liant à la molécule HLA de type B51.

L'invention concerne également le fragment polyépitopique de la protéine E6 de HPV tel que défini ci-dessus, caractérisé en ce qu'il correspond au fragment de 17 acides aminés délimité par les acides aminés situés aux positions 46 et 62, ou au fragment de 22 acides aminés délimité par les acides aminés situés aux positions 46 et 67 de la séquence peptidique de la protéine E6 de HPV, ce dernier fragment étant caractérisé par la séquence peptidique SEQ ID NO : 6 suivante :
(46)RREVYDFAFRDLCIVYRDGNPY(67)
ledit fragment contenant 6 épitopes se liant de façon stable à l'une au moins des 10 molécules HLA de types suivants : A2, A3, A11, A24, A29, B7, B27, B35, B44, ou B51, lesdits épitopes étant les suivants :
- (46)RREVYDFAFR(55) se liant de façon stable aux molécules HLA de type B27,
- (49)VYDFAFRDL(57) se liant de façon stable aux molécules HLA de type A24,
- (50)YDFAFRDL(57) se liant de façon stable aux molécules HLA de type A29, ou B44,
- (52)FAFRDLCIV(60) se liant de façon stable aux molécules HLA de type A2, B35, B51, ou B7,
- (54)FRDLCIVYR(62) se liant de façon stable aux molécules HLA de type A3, ou A11,
- (59)IVYRDGNPY(67) se liant de façon stable aux molécules HLA de type A3, ou A11.

L'invention concerne également les séquences peptidiques dérivées des fragments polyépitopiques susmentionnés de la protéine E6 (notamment ;
- par substitution, et/ou suppression, et/ou addition d'un ou plusieurs acides aminés, des fragments susmentionnés, et/ou
- par modification d'au moins une liaison peptidique -CO-NH- de la chaîne peptidique des fragments susmentionnés, notamment par introduction d'une liaison du type rétro, ou rétro-inverso, et/ou
- par substitution d'au moins un acide aminé de la chaîne peptidique de la séquence ou du fragment susmentionnés, par un acide aminé non protéinogénique,
lesdites séquences dérivées contenant des peptides ou pseudopeptides se liant spécifiquement à la ou aux mêmes molécules du CMH que celles se liant aux peptides contenus dans les fragments polyépitopiques susmentionnés dont elles dérivent.

Par séquence dérivée par introduction d'une liaison rétro-inverso, il faut entendre tout analogue peptidique d'un fragment susmentionné, ledit analogue étant constitué d'une chaîne peptidique dans laquelle l'un au moins des résidus d'une part est lié à au moins un résidu voisin par une liaison -NH-CO-, et d'autre part, est de chiralité opposée à celle de ce même résidu aminoacyle dans la chaîne peptidique du peptide parent (à savoir du fragment susmentionné dont elle dérive).

Par séquence dérivée par introduction d'une liaison rétro, il faut entendre tout analogue peptidique d'un fragment susmentionné, ledit analogue étant constitué d'une chaîne peptidique dans laquelle l'un au moins des résidus, est lié à au moins un résidu voisin par une liaison -NH-CO-, la chiralité de la totalité des résidus aminoacyles impliqués dans au moins une liaison -NH-CO- étant conservée par rapport aux résidus correspondant de la chaîne peptidique du peptide parent.

Il va de soi que les liaisons -CO-NH- et -NH-CO- doivent être prises en compte dans ce qui précède, dans le sens de la chaîne peptidique parente allant de l'extrémité aminoterminale (N-terminale) vers l'extrémité carboxyterminale (C-terminale).

Par "acide aminé protéinogénique", on entend, dans ce qui précède, tout acide aminé entrant dans la constitution d'une protéine ou d'un peptide naturel.

Par "acide aminé non protéinogénique", on entend par opposition à la définition précédente, tout acide aminé n'entrant pas dans la constitution d'une protéine ou d'un peptide naturel. On entend plus particulièrement par "acide aminé non protéinogénique", tout acide aminé dont le carbone portant la chaîne latérale R, à savoir le groupe -CHR-, situé entre -CO- et -NH- dans la chaîne peptidique naturelle, est remplacé par un motif n'entrant pas dans la constitution d'une protéine ou d'un peptide naturel.

L'invention a plus particulièrement pour objet les séquences dérivées telles que décrites ci-dessus, caractérisés en ce que l'une au moins des liaisons peptidiques -CO-NH- de la chaîne peptidique du peptide parent est remplacée par une liaison différente de la liaison -CO-NH-, ladite liaison différente étant notamment choisie parmi les suivantes :

| | |
|---|---|
| -CH₂-NH- | (méthylène amino) ; |
| -CH₂-CH₂- | (carba) ; |
| -CO-CH₂- | (cétométhylène) ; |
| -CH₂-O- | (méthylène-oxy) ; |
| -CHOH-CH₂- | (hydroxyéthylène) ; |
| -CHOH-CHOH- | (di-hydroxyéthylène) ; |
| -CH=CH- | (E ou Z oléfine) ; |
| -CHCN-NH- | (cyanométhylène amino) ; |
| -S-CH2- | (thiométhylène) ; |
| -CH₂-S- | (méthylène thio) ; |
| -CS-NH- | (thioamide) ; |
| -PO₂-NH- | (phosphonamide) ; |
| -CHOH- | (hydroxyméthylène) ; |
| -NH-CO-NH- | (urée) ; |
| 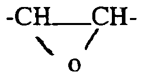 | (oxirane) ; |
| 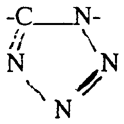 | (tétrazole) ; |
| -CH₂-CO-NH- | (β-homologation) ; |
| -CHOH-CH₂-NH- | (hydroxyéthylène amino) ; |
| -CO-NH-NH- | (hydrazino). |

L'invention a également pour objet les séquences nucléotidiques codant pour un fragment polyépitopique de la protéine E6, ou pour une séquence peptidique dérivée, tels que définis ci-dessus, lesdites séquences nucléotidiques étant issues de la séquence SEQ ID NO : 1 codant pour la protéine E6.

A ce titre, l'invention a plus particulièrement pour objet la séquence nucléotidique définie ci-dessous,
- la séquence SEQ ID NO : 5, codant pour le fragment polyépitopique SEQ ID NO : 6 susmentionné de la protéine E6,

L'invention a également pour objet tout vecteur, notamment plasmide, cosmide ou phage, contenant au moins une séquence nucléotidique susmentionnée placée sous le contrôle des éléments nécessaires à la transcription de ladite séquence, notamment sous le contrôle d'un promoteur et d'un terminateur de transcription.

L'invention concerne également les cellules hôtes, notamment bactéries, virus, levures, cellules eucaryotes, transformées à l'aide d'un vecteur susmentionné selon l'invention, de manière à intégrer de façon stable dans leur génome ou à maintenir de manière stable dans leur cytoplasme, au moins une séquence nucléotidique selon l'invention.

L'invention concerne également tout vecteur comprenant un ou plusieurs fragments polyépitopiques et/ou une ou plusieurs séquences peptidiques dérivées tels que définis ci-dessus, ou tout vecteur comprenant une ou plusieurs séquences nucléotidiques susmentionnées, lesdits vecteurs étant choisis parmi ceux capables d'assurer une protection desdits fragments ou séquences nucléotidiques dans l'organisme et/ou leur pénétration dans les cellules de l'organisme.

Dans le cas de l'utilisation de fragments polyépitopiques et/ou de séquences peptidiques dérivées susmentionnés, de tels vecteurs sont choisis parmi les acides gras (dans le cadre de la préparation de lipopeptides), les liposomes etc.

A ce titre, l'invention a plus particulièrement pour objet tout lipopeptide caractérisé en ce qu'il comprend:
- une partie peptidique comprenant un ou plusieurs fragments protéiques polyépitopiques choisis parmi ceux définis ci-dessus, ou toute séquence peptidique dérivée desdits fragments telle que définie ci-dessus,
- et une ou plusieurs parties lipophiles, avantageusement choisies parmi celles comprenant :
   * une chaîne hydrocarbonée en C4 à C20, saturée ou insaturée, linéaire ou ramifiée,
   * ou un groupe stéroïde, le cas échéant lié à la chaîne hydrocarbonée susmentionnée,
lesdites parties lipophiles étant éventuellement associées à un court peptide vecteur (pour former ainsi des motifs lipopeptidiques vecteurs) comportant une ou plusieurs fonctions ionisées à pH physiologique, et une fonction permettant la fixation covalente de ladite chaîne hydrocarbonée et/ou dudit groupe stéroïde.

Par partie lipophile, dans ce qui précède et ce qui suit, on entend toute molécule lipophile, insoluble dans l'eau, permettant, lorsqu'elle est liée à la partie peptidique définie ci-dessus, un passage intracellulaire passif du lipopeptide obtenu, grâce aux propriétés hydrophobes de ladite molécule. Avantageusement le lipopeptide résultant de la liaison de la partie lipophile à la partie peptidique, est soluble dans l'eau.

De préférence, la chaîne hydrocarbonée des parties lipophiles, est choisie parmi celles de :
- l'acide palmitique,
- l'acide oléique,
- l'acide linoléique,
- l'acide linolénique.

De préférence également, le groupe stéroïde de la ou des parties lipophiles, est choisi parmi les dérivés du cholestérol tel que l'acide cholest-5-ényl-3-oxy acétique, ou l'acide cholest-5-ényl-3-oxycarbonique.

L'invention a plus particulièrement pour objet tout lipopeptide tel que décrit ci-dessus, caractérisé en ce que la ou les parties lipophiles sont liées de façon covalente à un ou plusieurs acides aminés de la partie peptidique.

Avantageusement, la ou les parties lipophiles sont liées de façon covalente à la fonction αNH₂ ou *ε*NH₂ d'une lysine située en position N-terminale ou C-terminale de la partie peptidique, ou à la fonction thiol d'une cystéine, ou à toute fonction amino, alcool ou thiol éventuellement ajoutée au peptide avec un espaceur simple.

A ce titre, l'invention a plus particulièrement pour objet tout lipopeptide tel que défini ci-dessus, dans lequel la ou les parties lipophiles sont représentées par un groupe N^{α}-acétyl-Lysine N^{ε}(palmitoyl) (encore désigné par l'abréviation Ac-K(Pam)).

La présente invention a également pour objet, des micelles ou micro-agrégats d'un ou plusieurs lipopeptides différents définis ci-dessus.

Avantageusement, lesdits micelles ou micro-agrégats ont une taille inférieure à environ 1µm.

De préférence, les micelles ou micro-agrégats selon l'invention, sont tels qu'obtenus par dispersion desdits lipopeptides dans une solution d'acide acétique concentrée à environ 80%, ou tout autre solvant capable d'assurer une dispersion moléculaire des lipopeptides en solution.

Dans le cas de l'utilisation de séquences nucléotidiques définies ci-dessus selon l'invention, les vecteurs susmentionnés sont choisis parmi les virus, notamment les rétrovirus, les adénovirus et les virus associés (AAV Adeno Associated Virus).

L'invention a également pour objet les anticorps dirigés contre les fragments protéiques polyépitopiques ou les épitopes ou leurs séquences peptidiques dérivées (ou analogues) tels que définis ci-dessus, lesdits anticorps étant tels qu'obtenus par immunisation d'un animal avec au moins un des complexes susmentionnés, lesdits anticorps étant susceptibles de former un complexe avec ces fragments polyépitopiques ou ces épitopes ou leurs analogues.

Les anticorps selon l'invention sont des anticorps polyclonaux ou monoclonaux.

Les anticorps polyclonaux susmentionnés sont obtenus par immunisation d'un animal avec au moins un fragment protéique polyépitopique ou un épitope ou un analogue selon l'invention, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment un fragment protéique polyépitopique ou un épitope ou un analogue selon l'invention.

Les anticorps monoclonaux selon l'invention peuvent être obtenus par la technique des hybridomes dont le principe général est rappelé ci-après.

Dans un premier temps, on immunise un animal, généralement une souris, (ou des cellules en culture dans le cadre d'immunisations *in vitro)* avec un fragment protéique polyépitopique ou un épitope ou un analogue selon l'invention, contre lesquels les lymphocytes B de l'animal sont alors capables de produire des anticorps. Ces lymphocytes producteurs d'anticorps sont ensuite fusionnés avec des cellules myélomateuses "immortelles" (notamment murines) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clones, chacun conduisant à la production d'un anticorps monoclonal dont les propriétés de reconnaissance vis-à-vis du fragment protéique polyépitopique ou épitope ou analogue de l'invention pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

L'invention concerne également l'utilisation d'un ou plusieurs anticorps susmentionnés pour la mise en oeuvre d'une méthode de diagnostic *in vitro* des pathologies susmentionnées.

A ce titre l'invention a également pour objet des trousses ou kits comprenant lesdits anticorps, pour la mise en oeuvre d'une méthode de diagnostic telle que définie ci-dessus.

L'invention concerne également les compositions pharmaceutiques, ou vaccins, caractérisés en ce qu'ils comprennent :
* a)
   - au moins un fragment polyépitopique de la protéine E6 tel que défini ci-dessus,
   - et/ou au moins une séquence peptidique dérivée de ce fragment, telle que définie ci-dessus,
   - et/ou au moins un vecteur approprié, notamment des lipopeptides et/ou micelles définis ci-dessus, contenant au moins un fragment polyépitopique susmentionné de la protéine E6, et/ou au moins une séquence dérivée susmentionnée de ces fragments,
   en association avec un véhicule physiologiquement acceptable,
   ledit fragment protéique polyépitopique et/ou sa séquence dérivée étant le cas échéant associés à un ou plusieurs autres épitopes exogènes reconnus par des cellules T auxiliaires (encore désignés épitopes CD4 ou T helper), lesdits épitopes étant choisis notamment parmi les suivants :
   - le fragment peptidique délimité par les acides aminés situés aux positions 830 et 846 de la séquence peptidique de la toxine tétanique, ledit fragment répondant à la formule suivante : QYIKANSKFIGITELKK,
   - l'hémagglutinine (Prevost-Blondel et aL, 1995, J. Virol., 62, n°12, pp 8046-8055),
   - l'épitope PADRE (Alexander et al., 1994, Immunity, 1, 751).
* ou b)
   - au moins une séquence nucléotidique telle que définie ci-dessus, codant pour un fragment polyépitopique susmentionné de la protéine E6,
   - et/ou au moins une séquence nucléotidique codant pour une séquence peptidique dérivée de ce fragment, telle que définie ci-dessus,
      les séquences nucléotidiques susmentionnées pouvant être utilisées nues, en tant que minigènes,
   - et/ou au moins un vecteur approprié susmentionné, choisi notamment parmi les virus tels que définis ci-dessus, contenant au moins une séquence nucléotidique susmentionnée,
      en association avec un véhicule physiologiquement acceptable,
* ou c)
   - des anticorps définis ci-dessus, dirigés contre un fragment polyépitopique de la protéine E6, et/ou contre une séquence peptidique dérivée de ces fragments, tels que définis ci-dessus, en association avec un véhicule physiologiquement acceptable.

Avantageusement les compositions pharmaceutiques, ou vaccins, susmentionnés, se présentent sous une forme administrable par voie sous-cutanée, notamment à raison de plusieurs injections (avantageusement 3 injections) d'environ 500 µg du fragment polyépitopique sous forme lipopeptidique, à environ un mois d'intervalle.

L'invention a plus particulièrement pour objet l'utilisation de fragments polyépitopiques de la protéine E6 définis ci-dessus, ou de séquences peptidiques dérivées susmentionnées, ou de séquences nucléotidiques définies ci-dessus, ou d'anticorps susmentionnés, ou de lipopeptides définis ci-dessus, pour la préparation d'un médicament ou vaccin destiné à la prévention ou au traitement de pathologies liées à l'infection d'individus par les papillomavirus humains, telles que les néoplasies cervicales intraépithéliales (CIN), le cancer invasif du col de l'utérus, les néoplasies vulvaires intraépithéliales (VIN).

L'invention concerne également les peptides ou épitopes de la protéine E6 d'HPV choisis parmi les suivants :
- (46)RREVYDFAFR(55) se liant de façon stable aux molécules HLA de type B27,
- (49)VYDFAFRDL(57) se liant de façon stable aux molécules HLA de type A24,
- (50)YDFAFRDL(57) se liant de façon stable aux molécules HLA de type A29, B44,
- (52)FAFRDLCIV(60) se liant de façon stable aux molécules HLA de type A2, B35, B51,
- (54)FRDLCIVYR(62) se liant de façon stable aux molécules HLA de type A3, A11,
- (59)IVYRDGNPY(67) se liant de façon stable aux molécules HLA de type A3, A11,

L'invention concerne également les séquences peptidiques dérivées des peptides susmentionnés, lesdites séquences dérivées, ou analogues, étant telles que .définies ci-dessus dans le cadre des séquences dérivées des fragments protéiques polyépitopiques susmentionnés.

L'invention a également pour objet les séquences nucléotidiques codant pour les peptides de la protéine E6 susmentionnés, à savoir :
- la séquence délimitée par les nucléotides situés aux positions 136 et 165 de la séquence SEQ ID NO : 1, codant pour (46)RREVYDFAFR(55),
- la séquence délimitée par les nucléotides situés aux positions 145 et 171 de la séquence SEQ ID NO : 1, codant pour (49)VYDFAFRDL(57),
- la séquence délimitée par les nucléotides situés aux positions 148 et 171 de la séquence SEQ ID NO : 1, codant pour (50)YDFAFRDL(57),
- la séquence délimitée par les nucléotides situés aux positions 154 et 180 de la séquence SEQ ID NO : 1, codant pour (52)FAFRDLCIV(60),
- la séquence délimitée par les nucléotides situés aux positions 160 et 186 de la séquence SEQ ID NO : 1, codant pour (54)FRDLCIVYR(62),
- la séquence délimitée par les nucléotides situés aux positions 175 et 201 de la séquence SEQ ID NO : 1, codant pour (59)IVYRDGNPY(67),

L'invention a également pour objet tout procédé de préparation de fragments polyépitopiques, d'épitopes simples (peptides susmentionnés), ou de séquences dérivées, par synthèse peptidique classique en phase liquide ou en phase solide.

En variante, les fragments polyépitopiques, épitopes simples, ou séquences peptidiques dérivées, tels que définis ci-dessus selon l'invention, peuvent être obtenus sous forme de polypeptides recombinants par transformation de cellules hôtes appropriées telles que définies ci-dessus à l'aide de vecteurs contenant une séquence nucléotidique recombinante telle que définie ci-dessus selon l'invention, et récupération, le cas échéant après purification, du polypeptide recombinant codé par ladite séquence nucléotidique et produit par les cellules hôtes susmentionnées.

## Revendications

1. Utilisation de fragments polyépitopiques contenant 6 épitopes de la protéine E6 de HPV, choisis parmi ceux comprenant :
- le fragment contenant 6 épitopes délimité par les acides aminés situés aux positions 46 et 62, ou aux positions 46 et 67 de la séquence peptidique de la protéine E6, ce dernier fragment étant **caractérisé par** la séquence peptidique suivante:
(46)RREVYDF AFRDLCIVYRDGNPY(67)
ledit fragment contenant des peptides, chaque peptide se liant de façon stable à l'une au moins des molécules HLA de type A2, A3, A11, A24, A29, B7, B27, B35, B44, ou B51, lesdits épitopes étant les suivants :
- (46)RREVYDFAFR(55) se liant de façon stable aux molécules HLA de type B27,
- (49)VYDFAFRDL(57) se liant de façon stable aux molécules HLA de type A24,
- (50)YDFAFRDL(57) se liant de façon stable aux molécules HLA de type A29, ou B44,
- (52)FAFRDLCIV(60) se liant de façon stable aux molécules HLA de type A2, B35, B51, ou B7;
- (54)FRDLCIVYR(62) se liant de façon stable aux molécules HLA de type A3, ou A11,
- (59)IVYRDGNPY(67) se liant de façon stable aux molécules HLA de type A3, ou A11
et les séquences peptidiques dérivées des fragments polyépitopiques susmentionnés,
- par substitution et/ou addition d'un ou plusieurs acides aminés, des fragments susmentionnés, et/ou
- par modification d'au moins une liaison peptidique -CO-NH- de la chaîne peptidique des fragments susmentionnés, notamment par introduction d'une liaison du type rétro, ou rétro-inverso, et/ou
- par substitution d'au moins un acide aminé de la chaîne peptidique de la séquence ou du fragment susmentionné, par un acide aminé non protéinogénique, lesdites séquences dérivées contenant des peptides ou pseudopeptides, chaque peptide se liant spécifiquement à la ou aux mêmes molécules du CMH que celles se liant aux peptides contenus dans les fragments polyépitopiques susmentionnés dont elles dérivent, pour la préparation d'un médicament ou vaccin destiné à la prévention ou au traitement de pathologies liées à l'infection d'individus par les papillomavirus humains; telles que les néoplasies cervicales intraépithéliales (CIN), le cancer invasif du col de l'utérus, les néoplasies vulvaires intraépithéliales (VIN).

2. Utilisation de fragments polyépitopiques selon la revendication 1 **caractérisée en ce que** le nombre d'acides aminés de la séquence peptidique desdits fragments est supérieur ou égal à 17 et inférieur ou égal à 30.

3. Utilisation de fragments polyépitopiques de la protéine E6 de HPV selon l'une quelconque des revendications 1 ou 2, **caractérisée en ce que** ledit fragment contient 6 épitopes, chaque épitope se liant de façon stable à l'une au moins des 10 molécules HLA de types suivants: A2, A3, A11, A24, A29, B7, B27, B35, B44, ou B51, lesdits épitopes étant les suivants :
- (46)RREVYDFAFR(55) se liant de façon stable aux molécules HLA de type B27,
- (49)VYDFAFRDL(57) se liant de façon stable aux molécules HLA de type A24,
- (50)YDFAFRDL(57) se liant de façon stable aux molécules HLA de type A29, ou B44,
- (52)FAFRDLCIV(60) se liant de façon stable aux molécules HLA de type A2, B35, B51, ou B7;
- (54)FRDLCIVYR(62) se liant de façon stable aux molécules HLA de type A3, ou A11,
- (59)IVYRDGNPY(67) se liant de façon stable aux molécules HLA de type A3, ou A11.

4. Fragments polyépitopiques contenant 6 épitopes de la protéine E6 de HPV, choisis parmi ceux comprenant :
- le fragment contenant 6 épitopes délimité par les acides aminés situés aux positions 46 et 62, ou aux positions 46 et 67 de la séquence peptidique de la protéine E6, ce dernier fragment étant **caractérisé par** la séquence peptidique suivante:
(46)RREVYDFAFRDLCIVYRDGNPY(67)
ledit fragment contenant des peptides, chaque peptide se liant de façon stable à l'une au moins des molécules HLA de type A2, A3, A11, A24, A29, B7, B27, B35, B44, ou B51, et
- les séquences peptidiques dérivées des fragments polyépitopiques susmentionnés,
- par substitution et/ou addition d'un ou plusieurs acides aminés, des fragments susmentionnés, et/ou
- par modification d'au moins une liaison peptidique -CO-NH- de la chaîne peptidique des fragments susmentionnés, notamment par introduction d'une liaison du type rétro, ou rétro-inverso, et/ou
- par substitution d'au moins un acide aminé de la chaîne peptidique de la séquence ou du fragment susmentionnés, par un acide aminé non protéinogénique, lesdites séquences dérivées contenant des peptides ou pseudopeptides se liant spécifiquement à la ou aux mêmes molécules du CMH que celles se liant aux peptides contenus dans les fragments polyépitopiques susmenttionnés dont elles dérivent.

5. Fragments polyépitopiques selon la revendication 4 **caractérisés en ce que** le nombre d'acides aminés de la séquence peptidique desdits fragments est supérieur ou égal à 17 et inférieur ou égal à 30.

6. Fragments polyépitopiques de la protéine E6 de HPV selon l'une quelconque des revendications 4 ou 5, ledit fragment contenant 6 épitopes, chaque épitope se liant de façon stable à l'une au moins des 10 molécules HLA de types suivants : A2, A3, A11, A24, A29, B7, B27, B35, B44, ou B51, lesdits épitopes étant les suivants :
- (46)RREVYDFAFR(55) se liant de façon stable aux molécules HLA de type B27,
- (49)VYDFAFRDL(57) se liant de façon stable aux molécules HLA de type A24,
- (50)YDF AFRDL(57) se liant de façon stable aux molécules HLA de type A29, ou B44,
- (52)FAFRDLCIV(60) se liant de façon stable aux molécules HLA de type A2, B35, B51, ou B7,
- (54)FRDLCIVYR(62) se liant de façon stable aux molécules HLA de type A3, ou A11,
- (59)IVYRDGNPY(67) se liant de façon stable aux molécules HLA de type A3, ou A11.

7. Séquences nucléotidiques codant pour un fragment polyépitopique ou pour une séquence peptidique dérivée selon la revendication 4.

8. Anticorps, polyclonaux ou monoclonaux, dirigés contre un fragment polyépitopique ou contre une séquence peptidique dérivée selon la revendication 4.

9. Composition pharmaceutique, ou vaccin, **caractérisé en ce qu'**ils comprennent :
a)
- au moins un fragment polyépitopique de la protéine E6 défini dans la revendication 4,
- et/ou au moins une séquence peptidique dérivée de ce fragment, telle que définie dans la revendication 4,
- et/ou au moins un vecteur approprié, notamment des lipopeptides et/ou micelles, contenant au moins un fragment polyépitopique susmentionné de la protéine E6, et/ou au moins une séquence dérivée susmentionnée de ces fragments, en association avec un véhicule physiologiquement acceptable, ledit fragment protéique polyépitopique et/ou sa séquence dérivée étant le cas échéant associés à un ou plusieurs autres épitopes exogènes reconnus par des cellules T auxiliaires, tels que le fragment peptidique délimité par les acides aminés situés aux positions 830 et 846 de la séquence peptidique de la toxine tétanique, l'hémagglutinine, ou l'épitope PADRE,
ou b)
- au moins une séquence nucléotidique selon la revendication 7, codant pour un fragment polyépitopique susmentionné de la protéine E6,
- et/ou au moins une séquence nucléotidique codant pour une séquence peptidique dérivée de ce fragment, telle que définie ci-dessus,
- et/ou au moins un vecteur approprié susmentionné, choisi notamment parmi les virus, contenant au moins une séquence nucléotidique susmentionnée,
en association avec un véhicule physiologiquement acceptable,
ou c)
- des anticorps selon la revendication 8, dirigés contre un fragment polyépitopique de la protéine E6, et/ou contre une séquence peptidique dérivée de ces fragments, tels que définis ci-dessus.

10. Epitopes de la protéine E6 d'HPV choisis parmi les suivants :
- (46)RREVYDFAFR(55) se liant de façon stable aux molécules HLA de type B27,
- (50)YDFAFRDL(57) se liant de façon stable aux molécules HLA de type A29, B44,
- (54)FRDLCIVYR(62) se liant de façon stable aux molécules HLA de type A3, A11.

## Claims

1. Use of polyepitope fragments containing 6 epitopes of the HPV E6 protein, selected from those comprising:
- the fragment containing 6 epitopes delimited by amino acids situated in positions 46 and 62 or in positions 46 and 67 of the peptide sequence of the E6 protein, this latter fragment being **characterised by** the following peptide sequence:
(46)RREVYDF AFRDLCIVYRDGNPY(67)
this fragment containing peptides, each peptide being linked in a stable way to at least one of the HLA molecules of type A2, A3, A11, A24, A29, B7, B27, B35, B44 or B51, these epitopes being as follows:
- (46)RREVYDFAFR(55) being linked in a stable way to HLA molecules of type B27,
- (49)VYDFAFRDL(57) being linked in a stable way to HLA molecules of type A24,
- (50)YDFAFRDL(57) being linked in a stable way to HLA molecules of type A29 or B44,
- (52)FAFRDLCIV(60) being linked in a stable way to HLA molecules of type A2, B35, B51 or B7;
- (54)FRDLCIVYR(62) being linked in a stable way to HLA molecules of type A3 or A11,
- (59)IVYRDGNPY(67) being linked in a stable way to HLA molecules of type A3 or A11
and the resulting peptide sequences of the polyepitope fragments indicated above
- by substitution and/or addition of one or several amino acids of the fragments indicated above and/or
- by modification of at least one -CO-NH- peptide link of the peptide chain of the fragments indicated above, particularly by introduction of a retro or retro-inverse type link, and/or
- by substitution of at least one amino acid of the peptide chain of the sequence or of the fragment indicated above by a non-proteinogenic acid, these resulting sequences containing peptides or pseudopeptides, each peptide being linked specifically to the same molecule or molecules of the MHC as those linked to the peptides contained in the polyepitope fragments indicated above, from which they result, for the preparation of a medicine or vaccine intended for the prevention or treatment of pathologies linked to the infection of individuals by human papillomaviruses, such as cervical intraepithelial neoplasia (CIN), invasive cancer of the neck of the uterus and vaginal intraepithelial neoplasia (VIN).

2. Use of polyepitope fragments according to claim 1, **characterised in that** the number of amino acids of the peptide sequence of these fragments is greater than or equal to 17 and less than or equal to 30.

3. Use of polyepitope fragments of the HPV E6 protein according to any one of claims 1 or 2, **characterised in that** this fragment contains 6 epitopes, each epitope being linked in a stable way to at least one of the 10 HLA molecules of the following types: A2, A3, A11, A24, A29, B7, B27, B35, B44 or B51, these epitopes being as follows:
- (46)RREVYDFAFR(55) being linked in a stable way to HLA molecules of type B27,
- (49)VYDFAFRDL(57) being linked in a stable way to HLA molecules of type A24,
- (50)YDFAFRDL(57) being linked in a stable way to HLA molecules of type A29 or B44,
- (52)FAFRDLCIV(60) being linked in a stable way to HLA molecules of type A2, B35, B51 or B7;
- (54)FRDLCIVYR(62) being linked in a stable way to HLA molecules of type A3 or A11,
- (59)IVYRDGNPY(67) being linked in a stable way to HLA molecules of type A3 or A11.

4. Polyepitope fragments containing 6 epitopes of the HPV E6 protein, selected from those comprising:
- the fragment containing 6 epitopes delimited by amino acids situated in positions 46 and 62 or in positions 46 and 67 of the peptide sequence of the E6 protein, this latter fragment being **characterised by** the following peptide sequence:
(46)RREVYDFAFRDLCIVYRDGNPY(67)
this fragment containing peptides, each peptide being linked in a stable way to at least one of the HLA molecules of type A2, A3, A11, A24, A29, B7, B27, B35, B44 or B51 and
- the resulting peptide sequences of the polyepitope fragments indicated above
- by substitution and/or addition of one of several amino acids of the fragments indicated above and/or
- by modification of at least one -CO-NH- peptide link of the peptide chain of the fragments indicated above, particularly by the introduction of a retro or retro-inverse type link, and/or
- by substitution of at least one amino acid of the peptide chain of the sequence or of the fragment indicated above by a non-proteinogenic amino acid, these resulting sequences containing peptides or pseudopeptides being linked specifically to the same molecule or molecules of the MHC as those linked to the peptides contained in the polyepitope fragments indicated above, from which they result.

5. Polyepitope fragments according to claim 4, **characterised in that** the number of amino acids of the peptide sequence of these fragments is greater than or equal to 17 and less than or equal to 30.

6. Polyepitope fragments of the HPV E6 protein according to any one of claims 4 or 5, this fragment containing 6 epitopes, each epitope being linked in a stable way to at least one of the 10 HLA molecules of the following types: A2, A3, A11, A24, A29, B7, B27, B35, B44 or B51, these epitopes being as follows:
- (46)RREVYDFAFR(55) being linked in a stable way to HLA molecules of type B27,
- (49)VYDFAFRDL(57) being linked in a stable way to HLA molecules of type A24,
- (50)YDF AFRDL(57) being linked in a stable way to HLA molecules of type A29 or B44,
- (52)FAFRDLCIV(60) being linked in a stable way to HLA molecules of type A2, B35, B51 or B7,
- (54)FRDLCIVYR(62) being linked in a stable way to HLA molecules of type A3 or A11,
- (59)IVYRDGNPY(67) being linked in a stable way to HLA molecules of type A3 or A11.

7. Nucleotide sequences coding for a polyepitope fragment or for a resulting peptide sequence according to claim 4.

8. Polyclonal or monoclonal antibodies directed against a polyepitope fragment or against a resulting peptide sequence according to claim 4.

9. Pharmaceutical composition or vaccine, **characterised in that** it comprises:
a)
- at least one polyepitope fragment of the E6 protein defined in claim 4
- and/or at least one resulting peptide sequence of this fragment, such as defined in claim 4,
- and/or at least one suitable vector, particularly lipopeptides and/or micelles,
containing at least one polyepitope fragment indicated above of the E6 protein and/or at least one resulting sequence of these fragments indicated above, in association with a physiologically acceptable vehicle, this polyepitope proteinic fragment and/or its resulting sequence, if necessary, being associated with one or several other exogenous epitopes recognised by T helper cells, such as the peptide fragment delimited by amino acids situated in positions 830 and 846 of the peptide sequence of tetanus toxin, hemagglutinin or the PADRE epitope,
or b)
- at least one nucleotide sequence according to claim 7, coding for a polyepitope fragment of the E6 protein indicated above,
- and/or at least one nucleotide sequence coding for a resulting peptide sequence of this fragment, such as defined above,
- and/or at least one suitable vector indicated above, selected particularly from the viruses, containing at least one nucleotide sequence indicated above,
in association with a physiologically acceptable vehicle,
or c)
- antibodies according to claim 8 directed against a polyepitope fragment of the E6 protein and/or against a resulting peptide sequence of these fragments, such as defined above.

10. Epitopes of the HPV E6 protein selected from the following:
- (46)RREVYDFAFR(55) being linked in a stable way to HLA molecules of type B27,
- (50)YDFAFRDL(57) being linked in a stable way to BOA molecules of type A29 and B44,
- (54)FRDLCIVYR(62) being linked in a stable way with BOA molecules of type A3 and A11.

## Patentansprüche

1. Verwendung von polyepitopischen Fragmenten, enthaltend 6 Epitope der Proteine E6 von HPV, ausgewählt aus solchen, die folgende Bruchstücke enthalten:
- das Fragment mit 6 Epitopen, begrenzt von den Aminosäuren, die sich an den Positionen 46 und 62 befinden, oder an den Positionen 46 und 67 der Peptidsequenz des Proteins E6, wobei letzteres Fragment von der folgenden Peptidsequenz gekennzeichnet ist:
(46)RREVYDFAFRDLCIVYRDGNPY(67),
wobei das genannte Fragment Peptide aufweist und jedes Peptid stabil an mindestens eines der Moleküle HLA des Typs A2, A3, A11, A24, A29, B7, B27, B35, B44 oder B51 gebunden ist und die genannten Epitope die folgenden sind:
- (46)RREVYDFAFR(55), welches stabil mit den Molekülen HLA des Typs B27 verbunden ist,
- (49)VYDFAFRDL(57), welches sich stabil mit den Molekülen HLA des Typs A24 verbindet,
- (50)YDFAFRDL(57), welches sich stabil mit den Molekülen HLA des Typs A29 oder B44 verbindet,
- (52)FAFRDLCIV(60), welches sich stabil an die Moleküle HLA des Typs A2, B35, B51 oder B7 bindet;
- (54)FRDLCIVYR(62), welches sich stabil an die Moleküle HLA des Typs A3 oder A11 bindet,
- (59)IVYRDGNPY(67), welches sich stabil an die Moleküle HLA des Typs A3 oder A11 bindet und die Peptidsequenzen, welche sich von den oben genannten polyepitopischen Fragmenten ableiten,
- durch Substitution und/oder Addition gegen eine bzw. an eine oder mehrere Aminosäuren für die oben genannten Fragmente und/oder
- durch Modifizierung mindestens einer Peptidbindung -CO-NH- der Peptidkette der oben genannten Fragmente, insbesondere durch Einführung einer Bindung des Typs Retro oder Retro-inverso,
- und/oder Substitution mindestens einer Aminosäure der Peptidkette der Sequenz oder des oben genannten Fragments durch eine nicht proteinogenetische Aminosäure, wobei die oben genannten abgeleiteten Sequenzen Peptide oder Pseudopeptide enthalten und jedes Peptid spezifisch mit dem oder den gleichen Molekülen des CMH wie diejenigen verbunden ist, welche sich mit den Peptiden verbinden, die sich in den oben genannten polyepitopischen Fragmenten befinden, von denen sie abgeleitet sind,
für die Herstellung eines Medikaments oder eines Impfstoffes zur Verhütung oder zur Behandlung von Krankheiten auf der Grundlage der Infektion von Personen durch den menschlichen Papillomavirus, beispielsweise der intraepithelialen Neoplasien des Gehirns (CIN), durch den Krebs des Gebärmutterhalses und der intraepithelialen vulvären Neoplastien (VIN).

2. Verwendung der polyepitopischen Fragmente nach Anspruch 1, **dadurch gekennzeichnet, dass** die Anzahl der Aminosäuren in der Peptidsequenz der genannten Fragmente gleich oder grösser als 17 und kleiner oder gleich 30 ist.

3. Verwendung von polyepitopischen Fragmenten des Proteins E6 der HPV nach einem der Ansprüche 1 und 2, **dadurch gekennzeichnet, dass** das genannte Fragment 6 Epitope enthält und sich jedes Epitop stabil mit mindestens einem der 10 Moleküle HLA des folgenden Typs verbindet: A2, A3, A11, A24, A29, B7, B27, B35, B44 oder B51, wobei die genannten Epitope die folgenden sind:
- (46) RREVYDFAFR (55), welches sich stabil mit den Molekülen HLA des Typs B27 verbindet,
- (49)VYDFAFRDL(57), welches sich stabil an die Moleküle HLA des Typs A24 bindet,
- (50)YDFAFRDL(57), welches sich stabil an die Moleküle HLA des Typs A29 oder B44 bindet,
- (52)FAFRDLCIV(60), welches sich stabil an die Moleküle HLA des Typs A2, B35, B51 oder B7 bindet,
- (54)FRDLCIVYR(62), welches sich stabil an die Moleküle HLA des Typs A3 oder A11 bindet,
- (59)IVYRDGNPY(67), welches sich stabil an die Moleküle HLA des Typs A3 oder A11 bindet.

4. Polyepitopische Fragmente mit 6 Epitopen des Proteins E6 von HPV, ausgewählt unter denjenigen, welche folgende Bausteine enthalten:
- das Fragment, welches 6 Epitope aufweist und von den Aminosäuren begrenzt wird, welche sich an den Positionen 46 und 62 oder den Positionen 46 und 67 der Peptidsequenz des Proteins E6 befinden, wobei das letztere Fragment durch die folgende Peptidsequenz charakterisiert wird:
(46) RREVYDFAFRDLCIVYRDGNPY (67)
und wobei dieses Fragment Peptide enthält und sich jedes dieser Peptide stabil mindestens an ein Molekül HLA des Typs A2, A3, A11, A24, A29, B7, B27, B35, B44 oder B51 bindet, und
- die Peptidsequenzen, welche sich von den oben genannten polyepitopischen Fragmenten ableiten,
- durch Substitution und/oder Addition einer oder mehrerer Aminosäuren der oben genannten Fragmente, und/oder
- durch Modifizierung mindestens einer Peptidbindung -CO-NH- der Peptidkette der oben genannten Fragmente, insbesondere durch Einführung einer Bindung des Typs Retro oder Retro-inverso, und/oder
- durch Substitution mindestens einer Aminosäure der Peptidkette der oben genannten Sequenz oder des genannten Fragments durch eine nicht proteinerzeugende Aminosäure, wobei die genannten abgeleiteten Sequenzen Peptide oder Pseudopeptide enthalten, die sich spezifisch an das oder die gleichen Moleküle des CMH wie diejenigen binden, die sich an die Peptide binden, welche in den oben genannten polyepitopischen Fragmenten enthalten sind, von denen sie sich ableiten.

5. Polyepitopische Fragmente nach Anspruch 4, **dadurch gekennzeichnet, dass** die Anzahl der Aminosäuren in der Peptidsequenz der genannten Fragmente grösser oder gleich 17 und gleich oder kleiner als 30 ist.

6. Polyepitopische Fragmente des Proteins E6 der HPV nach einem der Ansprüche 4 und 5, wobei dieses Fragment 6 Epitope aufweist und jedes Epitop eine stabile Verbindung mit mindestens einem der 10 Moleküle HLA des folgenden Typs eingeht: A2, A3, A11, A24, A29, B7, B27, B35, B44 oder B51 und die genannten Epitope die folgenden sind:
- (46) RREVYDFAFR (55), welches sich stabil mit den Molekülen HLA des Typs B27 verbindet,
- (49)VYDFAFRDL(57), welches sich stabil an die Moleküle HLA des Typs A24 bindet,
- (50)YDFAFRDL(57), welches sich stabil an die Moleküle HLA des Typs A29 oder B44 bindet,
- (52)FAFRDLCIV(60), welches sich stabil an die Moleküle HLA des Typs A2, B35, B51 oder B7 bindet,
- (54)FRDLCIVYR(62), welches sich stabil an die Moleküle HLA des Typs A3 oder A11 bindet,
- (59)IVYRDGNPY(67), welches sich stabil an die Moleküle HLA des Typs A3 oder A11 bindet.

7. Nukleotidsequenzen, die für ein polyepitopisches Fragment oder für eine Peptidsequenz kodieren, gemäss Anspruch 4.

8. Polyklonale oder monoklonale Antikörper, welche gegen ein polyepitopisches Fragment oder gegen eine abgeleitete Peptidsequenz gemäss Anspruch 4 gerichtet sind.

9. Pharmazeutische Zusammensetzung oder Impfstoff, **dadurch gekennzeichnet, dass** sie
a)
- mindestens ein polyepitopisches Fragment des Proteins E6 gemäss Anspruch 4
- und/oder mindestens eine von diesem Fragment abgeleitete Peptidsequenz gemäss Anspruch 4
- und/oder einen geeigneten Trägerstoff enthalten, insbesondere Lipopeptide und/oder Mizellen, welche mindestens ein oben genanntes polyepitopisches Fragment des Proteins E6 aufweisen, und/oder mindestens eine Sequenz, welche von den oben erwähnten Fragmenten abgeleitet ist, das Ganze in Verbindung mit einem physiologisch annehmbaren Trägerstoff, wobei das genannte polyepitopische Proteinfragment und/oder seine davon abgeleitete Sequenz gegebenenfalls zusammen mit einem oder mehreren exogenen anderen Epitopen kombiniert sind, welche von den Hilfs-T-Zellen erkannt werden, beispielsweise das Peptidfragment, welches an den Positionen 830 und 846 der Peptidsequenz des Tetanustoxins, des Hämaglutinins oder des Epitops PADRE erkannt werden,
oder b)
- mindestens eine Nukleotidsequenz nach Anspruch 7, welche für ein oben bezeichnetes polyepitopisches Fragment des Proteins E6 kodiert,
- und/oder mindestens eine Nukleotidsequenz, welche für eine Peptidsequenz kodiert, die von diesem Fragment abgeleitet und wie oben definiert ist,
- und/oder mindestens einen oben genannten geeigneten Trägerstoff, welcher insbesondere aus den Viren ausgewählt ist, welche mindestens eine oben genannte Nukleotidsequenz aufweise, in Verbindung mit einem physiologisch akzeptablen Trägerstoff,
oder c)
- die in Anspruch 8 genannten Antikörper, die gegen ein polyepitopisches Fragment des Proteins E6 gerichtet sind, und/oder gegen eine von diesen Fragmenten abgeleitete Peptidsequenz gemäss obiger Definition.

10. Epitope des Proteins E6 von HPV, ausgewählt aus den folgenden:
- (46) RREVYDFAFR (55), welches sich stabil mit den Molekülen HLA des Typs B27 verbindet,
- (50)YDFAFRDL(57), welches sich stabil an die Moleküle HLA des Typs A29, B44 bindet,
- (54)FRDLCIVYR(62), welches sich stabil an die Moleküle HLA des Typs A3, A11 bindet.
